# EUROPEAN PATENT SPECIFICATION

(11) **EP 2 420 508 B1**
(45) Date of publication and mention of the grant of the patent: **23.09.2015**
(21) Application number: 11177252.1
(22) Date of filing: 11.08.2011
(51) Int. Cl.: C07H 19/20

(54) **Modified nucleotide and real-time polymerase reaction using the same**
Modifiziertes Nukleotid und dessen Verwendung in der Echtzeit-Polymerasereaktion
Nucléotide modifié et réaction de polymérase en temps réel l'utilisant

(30) Priority: 16.08.2010 KR 20100078926
(43) Date of publication of application: 22.02.2012
(73) Proprietor: Korea Institute of Science and Technology, Seoul 136-791 (KR)
(72) Inventor: Ahn, Dae-Ro, 413-735 Gyeonggi-do (KR)
(74) Representative: advotec.

(56) References cited:
- WO-A2-2009/014612
- BAMBI REYNOLDS ET AL: "Synthesis and Stability of Novel Terminal Phosphate-Labeled Nucleotides", NUCLEOSIDES, NUCLEOTIDES & NUCLEIC ACIDS, vol. 27, no. 1, 1 January 2008 (2008-01-01), pages 18-30, XP55009225, ISSN: 1525-7770, DOI: 10.1080/15257770701571768
- KUMAR SHIV ET AL: "TERMINAL PHOSPHATE LABELED NUCLEOTIDES: SYNTHESIS, APPLICATIONS, AND LINKER EFFECT ON INCORPORATION BY DNA POLYMERASES", NUCLEOSIDES, NUCLEOTIDES AND NUCLEIC ACIDS, TAYLOR & FRANCIS, PHILADELPHIA, PA, vol. 24, no. 5-7, 1 January 2005 (2005-01-01), pages 401-408, XP009075271, ISSN: 1525-7770, DOI: 10.1081/NCN-200059823
- DA-RAE KIM ET AL: "A novel real-time PCR method based on signaling-by-incorporation", CHEMICAL COMMUNICATIONS, vol. 47, no. 2, 1 January 2011 (2011-01-01), page 791, XP55009227, ISSN: 1359-7345, DOI: 10.1039/c0cc04516k

## Description

### BACKGROUND OF THE INVENTION

### (a) Field of the Invention

The present invention relates to a modified nucleotide and real-time polymerase reaction using the nucleotide. Specifically, the present invention relates to a fluorescence material linked-nucleotide, a composition for real-time polymerase reaction comprising the nucleotide, an analysis kit, and an analysis method.

### (b) Description of the Related Art

Quantitative analysis of DNA polymerase reaction is an essential step applied to diverse techniques such as when measuring a particular gene expression, calculating a particular gene concentration in a sample and performing immuno-PCR by using DNA-antibody conjugates.

Such quantitative analysis requires a method that detects a signal showing DNA polymerase reaction progression since DNA itself does not possess a signal producing region.

A conventional method of staining DNA with intercalating dyes between DNA bases after gel-electrophoresis has been widely used. Owing to its complexity, the method, however, is impossible to be automated and has a very narrow dynamic range of DNA detection. Furthermore, the real-time quantitative measurement of DNA contents produced during polymerization reaction is impossible, because the method includes the use of a gel, which only allows end-point detection of DNA contents.

In order to overcome such drawbacks, two types of probe molecules for reporting fluorescence signals are currently used to analyze real-time polymerase reaction.

One method utilizes DNA-bound fluorescent dye (SYBR green I). Although the dye cannot emit fluorescence by itself, the dye emits fluorescence at the time of binding with DNA. The binding of dye with DNA largely depends on the amount of DNA duplexes produced during DNA polymerase reaction. Therefore, this method allows the detection of fluorescence intensity proportional to the DNA duplexes amount.

Alternatively, the other method uses oligonucleotide labeled with a fluorophore and a quencher at its 5', 3' terminal. The oligonucleotide sequence is complementary with a part of the DNA sequence produced during DNA polymerase reaction. When the oligonucleotide is added to a polymerase reaction solution, the polymerase binds to the complementary region and the oligonucleotide may be structurally modified (hybridization probe) or destructed by activation of nuclease (hydrolysis probe: TaqMan probe) so that the fluorescence intensity may increase. Therefore, fluorescence intensity increases proportional to DNA amount produced by the reaction and this signal allows the quantitative real-time analysis of the polymerase reaction.

However, the methods described above have many drawbacks.

First, the method using the DNA-bound fluorescent dye cannot be applied to a single strand produced by polymerase reaction such as RCA (rolling circle amplification) because the dye often negatively affects polymerase reaction efficiency and has a tendency to bind more readily to a DNA duplex than a single strand DNA. (Gusev, Y et al. American Journal of Pathology, 159, 63-69, 2001) The method may also produce a false-positive signal because a fluorescence signal continues to be emitted even when the dye non-specifically binds to a double strand such as a primer dimer which is produced regardless of polymerase reaction. (Ponche, F et al. BMC Biotechnology, 3, 18, 2003)

Similarly, in addition to its high production costs for the probe, the method using the oligonucleotide has a disadvantage in that the probe has to be designed with a careful selection of a nucleotide sequence to obtain a desired detection signal. Particularly, the method is not reliable to analyze the polymerase reaction under isothermal reaction condition, because the oligonucleotide probe molecule is difficult to bind the reaction product, and even if it binds, it requires extremely long period of time due to thermal stability of the double strand produced. Such disadvantage seems to be caused by additionally requiring a probe just for the purpose of a signal detection, when it itself does not directly participate in the polymerase reaction.

### SUMMARY OF THE INVENTION

In order to overcome such drawbacks mentioned above, the present inventors have developed a method for analyzing real-time polymerase reaction using a modified nucleotide without requiring a probe. The modified nucleotide can be obtained by linking a fluorescence material with a chemically modified dGTP or dATP, having a purine base among nucleotide monomers directly participating in real-time polymerization reaction.

Thus, an embodiment provides a composition for real-time polymerase reaction comprising a fluorescence material linked-nucleotide having Formula (1) as shown below.

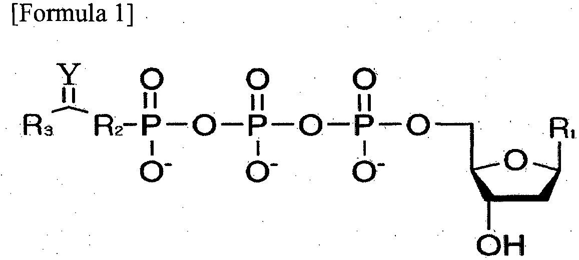

Another embodiment provides an analysis kit for real-time polymerase reaction comprising the composition for real-time polymerase reaction.

Still another embodiment provides a method for analyzing real-time polymerase reaction comprising the steps of (a) providing the composition for real-time polymerase reaction and primers capable of amplifying a region of target nucleic acid, (b) extracting nucleic acids from samples and performing real-time polymerase reaction by using the composition and the primers provided in step (a), and (c) analyzing target nucleic acid content in the sample by measuring fluorescence signal in step (b).

### DETAILED DESCRIPTION OF THE EMBODIMENTS

In order to achieve the objectives mentioned above, an embodiment provides a composition for a real-time polymerase reaction comprising a fluorescence material linked-nucleotide having Formula (1) below.

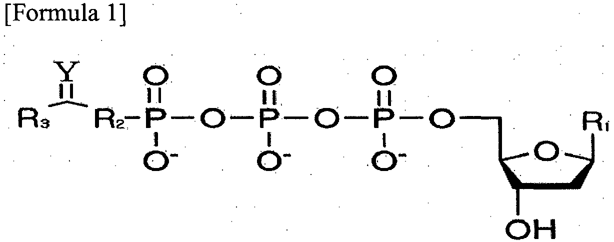

Also, another embodiment provides an analysis kit for real-time polymerase reaction comprising the composition.
Also, another embodiment provides a method for analyzing real-time polymerase reaction comprising the steps of (a) preparing the composition for real-time polymerase reaction and primers capable of amplifying a region of target nucleic acid, (b) extracting nucleic acids from samples and performing real-time polymerase reaction by using the composition and the primers prepared in step (a), and (c) analyzing target nucleic acid content by measuring fluorescence signal in step (b).

The present invention will be explained in more detail below.

The fluorescence material linked-nucleotide is labeled with a fluorescence material at the γ-phosphate group of the nucleotide via a linker. Therefore, the fluorescence material linked-nucleotide comprises a fluorescence material and a purine base, wherein the purine base plays a role in quenching fluorescence in the nucleotide.

The fluorescence material linked-nucleotide is represented by the following Formula 1

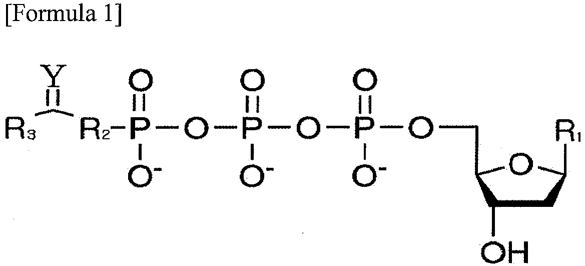

In Formula 1, R₁ is an adenine or guanine, R₂ is -(CH₂OCH₂)m-CH₂NH-, -NHCH₂-(CH₂OCH₂)m-CH₂NH-, -(CH₂)n-NH-, or -NH-(CH₂)n-NH-; m is an integer from 1 to 20; n is an integer from 2 to 60; R₃ is a fluorescence material; and Y is O or S.

The fluorescence material linked-nucleotide is labeled with a fluorescence material(R₃) at the γ-phosphate group of the nucleotide via a linker(R₂), wherein the nucleotide is dATP (deoxyadenosine triphosphate) or dGTP (deoxyguanosine triphosphate) having the purine base as shown above. The linker is -(CH₂OCH₂)m-CH₂NH-, -NHCH₂-(CH₂OCH₂)m-CH₂NH-, -(CH₂)n-NH- or -NH-(CH₂)n-NH-, wherein m is an integer from 1 to 20 and n is integer from 2 to 60. If m is more than 20 or n is more than 60, effective fluorescence quenching may not be observed since the distance between the purine-base responsible for fluorescence quenching and the fluorescence material is too far apart. If m is less than 1 or n is less than 2, the fluorescence material linked-nucleotide may not be used as a polymerase-substrate. More preferably, m may be an integer from 2 to 10 and n may be an integer from 2 to 20.

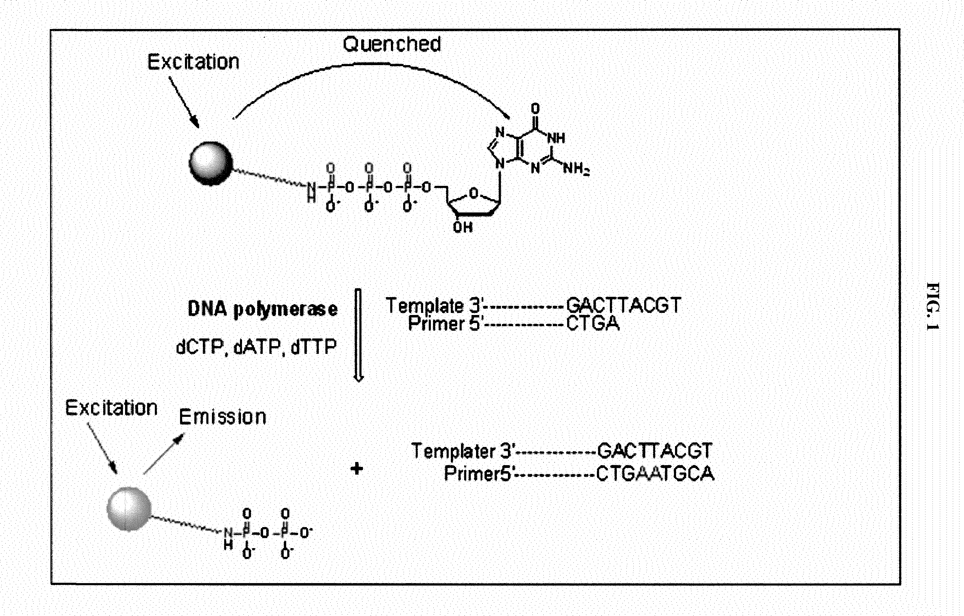

The fluorescence material linked-nucleotide is characterized by comprising the purine base responsible for fluorescence quenching in the nucleotide and the fluorescence material. Particularly, if the fluorescence material linked-nucleotide is used as a substrate by DNA polymerase, a pyrophosphate labeled by the fluorescence material may be formed as a byproduct. Then, fluorescence intensity quenched prior to polymerase reaction may be restored and increase. (see Fig. 1)

In the conventional real-time polymerase analysis method, probes producing fluorescence signal have been considered as an essential material in addition to a substrate (dNTPs). However, in case of fluorescence material linked-nucleotide of the present invention, the probes are not necessary other than the substrates (dNTPs), and fluorescence signal can be produced during the polymerase reaction itself.

The fluorescence material may be a material where its fluorescence intensity can be quenched by a purine base. The fluorescence material may include, but is not limited to, a material selected from group consisting of fluorescein, Bodipy-FL, Bodipy-R6G, Pacific Blue, Marina Blue, coumarin, tetramethylrhodamine, Cy5, Cy3 and Texas Red. Most preferably, the fluorescence material may be Bodipy-FL where its fluorescence intensity can be quenched most effectively by a purine base.

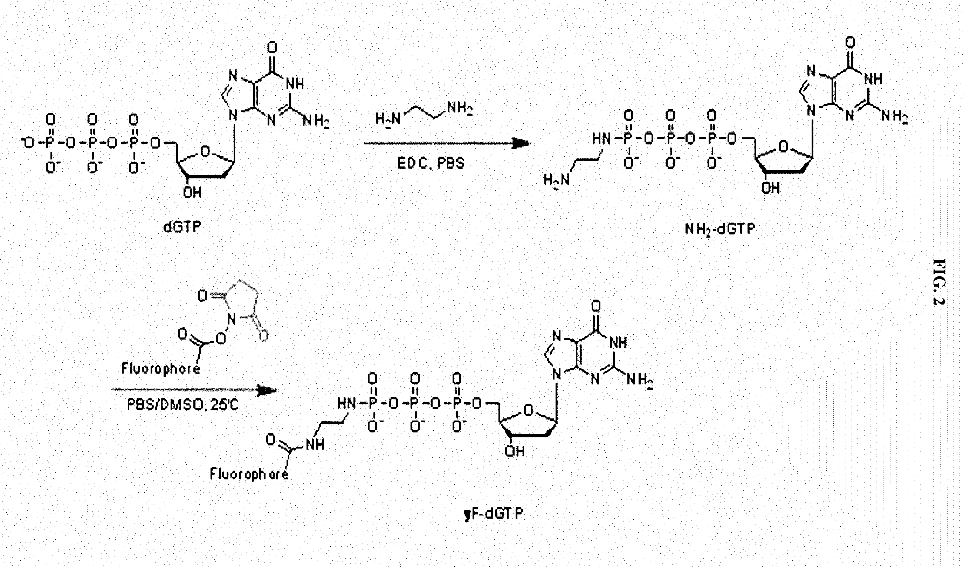

The fluorescence material linked-nucleotide can be prepared by the following steps.
(1) a step of forming amine group at the γ-phosphate group of dATP or dGTP by reacting dATP or dGTP and amine, preferably ethylenediamine, having a strand consisting of from 2 to 60 elements, wherein the element is carbon or carbon/oxygen.
(2) a step of reacting dATP or dGTP with a fluorescent substance, wherein the dATP or dGTP includes amine group formed at the γ-phosphate group and the fluorescent substance is linked with succinimidyl ester group. (see Fig. 2)

The composition for real-time polymerase reaction is characterized by comprising the fluorescence material linked-nucleotide, but is not limited thereto.

The composition for real-time polymerase reaction may be a reaction solution comprising a substrate (dNTPs) used for real-time polymerase reaction. The composition is characterized by comprising the fluorescence material linked-nucleotide instead of the conventional dATP or dGTP as a substrate.

The composition for real-time polymerase reaction may further comprise, but is not limited to, dCTP (deoxycytidine triphosphate) and dTTP (deoxythymidine triphosphate).

The composition for real-time polymerase reaction may further comprise a polymerase capable of using the fluorescence material linked-nucleotide as a substrate, wherein the polymerase may be, but is not limited to, selected from group consisting of Taq DNA polymerase, Therminator γ DNA polymerase and phi29 DNA polymerase

The taq DNA polymerase may be, but is not limited to, polymerase having AAA27507 as NCBI Accession No. The therminator γ DNA polymerase may be, but is not limited to, purchased from New England Biolabs (catalog no: M0334L or M0334S). The phi29 DNA polymerase may be, but is not limited to, polymerase having YP_002004529 as NCBI Accession No.

The composition for real-time polymerase reaction may further comprise a buffer solution and a salt necessary for polymerase reaction that are well known to the person skilled in the arts. The buffer solution and salt may be, but are not limited, ones commonly used for DNA polymerase reaction.

Moreover, the composition for real-time polymerase reaction may be used to quantitatively analyze a target nucleic acid by performing polymerase reaction with primers capable of amplifying a specific region of the target nucleic acid, which is used as a template.

The target nucleic acid refers to a nucleic acid sequence of interest to an ordinary skilled person. If a target nucleic acid sequence is determined, the skilled person can easily design primers capable of specifically amplifying the target nucleic acid sequence.

In the present invention, a real-time polymerase reaction may be a reaction capable of determining the presence of the target nucleic acid by specifically copying and amplifying a specific region of nucleic acid (DNA or RNA). Furthermore, a real-time polymerase reaction may be one capable of quantitatively analyzing a target nucleic acid. The real-time polymerase reaction may be, but is not limited to, selected from a group consisting of real-time polymerase chain reaction (PCR), isothermal polymerization and real-time rolling circle amplification (RCA).

Particularly, the real-time PCR may be a method for quantitatively analyzing a target nucleic acid in a sample comprising the following steps:
(a) a step of measuring fluorescence intensity according to a target nucleic acid concentration change by amplifying the target nucleic acid with the composition of the present invention, using a target nucleic acid and primers capable of specifically amplifying the target nucleic acid, wherein the target nucleic acid concentration is known before measuring fluorescence intensity.
(b) a step of analyzing PCR reaction cycles (Ct value) required to reach a certain threshold of fluorescence intensity.
(c) a step of drawing a standard curve of the target nucleic acid concentration against corresponding Ct value.
(d) a step of extracting a nucleic acid from a sample, amplifying the nucleic acid by PCR and obtaining a Ct value.
(e) a step of quantitatively analyzing the target nucleic acid included in the sample by matching the Ct value with standard curve.

The composition for real-time polymerase reaction of the present invention can solve a problem associated with using conventional DNA-fluorescent dyes. Particularly, in case of performing PCR analysis with the conventional DNA-fluorescent dye (SYBR green I), the dye cannot be utilized in the analysis if a single strand such as RCA is a product of polymerase reaction because the DNA-fluorescent dye has a tendency to bind to a DNA duplex more readily than a single strand. However, the composition for real-time polymerase reaction of the present invention can be applied to RCA.

Moreover, the composition for real-time polymerase reaction does not require probes which had been considered to be an essential component in the conventional real-time polymerase reaction. Therefore, the composition of the present invention can reduce both cost and time required for designing probes by selecting a specific nucleic acid sequence to produce a detection signal.

Meanwhile, the analysis kit for real-time polymerase reaction is characterized by comprising the composition for real-time polymerase reaction.

The analysis kit may further comprise components well known in the art as well as the composition for real-time polymerase reaction. For example, the analysis kit may further comprise a material such as a bead and a matrix.

The analysis kit may be used for real-time polymerase reaction selected from a group consisting of real-time polymerase chain reaction (PCR), isothermal polymerization and real-time rolling circle amplification (RCA). Furthermore, the analysis kit may be used for real-time RT (reverse transcription)-PCR, with a proviso that a detection target is RNA.

A fluorescence signal emitted by using the analysis kit can be measured by a device well known in the art.

Meanwhile, the method for analyzing real-time polymerase reaction is characterized by comprising the steps of;
(a) preparing the composition for real-time polymerase reaction and primers capable of amplifying a region of target nucleic acid;
(b) extracting nucleic acids from samples and performing real-time polymerase reaction by using the composition and the primers prepared in step (a); and
(c) analyzing target nucleic acid content by measuring fluorescence signal in step (b).

The method for analyzing real-time polymerase reaction may be a method capable of determining the presence of a target nucleic acid by specifically copying and amplifying a specific region of nucleic acid (DNA or RNA). Furthermore, the inventive method may be one capable of quantitatively detecting the target nucleic acid based on real-time. Preferably, the method may be one capable of determining the presence of a target nucleic acid and quantitatively detecting the target nucleic acid on real-time basis by measuring fluorescence intensity from real-time polymerase reaction.

Each step will be explained in more detail as follows.

In the step (a), the primers can amplify a specific region of the target nucleic acid. If the specific region of target nucleic acid is determined, an ordinary skilled person can easily prepare the primers according to the method well known in the art.

In the step (b), the sample may preferably be blood fluid, blood serum, blood plasma or blood from animals including human. An ordinary skilled person can extract nucleic acid from the sample according to the method well known in the art, and more preferably can extract according to the manual specified in QIAamp DNA Blood Mini Kit (Qiagne).

In the step (b), as mentioned above, it is preferable to use a polymerase capable of using the fluorescence material linked-nucleotide as a substrate when performing real-time polymerase reaction with the primers and the composition of the present invention, and using a targeted nucleic acid extracted from the sample as a template.

In the step (c), the fluorescence signal may be measured by using a device for measuring fluorescence signal well known in the art. The content of nucleic acid from the sample may be quantitatively analyzed based on the measurement results.

Particularly, quantitative analysis using the method of the present invention may be performed by the following steps.
(a) a step of measuring fluorescence intensity according to a target nucleic acid concentration change by amplifying a target nucleic acid with the composition of the present invention, using a target nucleic acid and primers capable of specifically amplifying the target nucleic acid, wherein the target nucleic acid concentration is known before measuring fluorescence intensity.
(b) a step of analyzing PCR reaction cycles (Ct value) required to reach a certain threshold.
(c) a step of drawing a standard curve of the target nucleic acid concentration against corresponding Ct value.

Ordinary skilled person can readily perform the quantitative analysis by using the standard curve.

The method of the present invention may be used for real-time polymerase reaction selected from a group consisting of real-time polymerase chain reaction (PCR), isothermal polymerization and real-time rolling circle amplification (RCA), but is not limited thereto. Furthermore, the method may be used for real-time RT (reverse transcription)-PCR, with a proviso that a detection target is RNA.

The method of the present invention may be more effectively used to analyze real-time polymerase reaction as the comparative items showed improved results in terms of efficiency or sensitivity when compared to the conventional method using SYBR green I. (see table 2)

In addition, the method is impossible to emit an incorrect signal caused by probes as in the past method, and economically advantageous. Particularly, as shown in Table 1 below, the method may be more diversely applied because the analysis can be performed in much more simple manner compared to the conventional methods..

**[Table 1]**

| Comparison the present invention with the past method | | | | |
|---|---|---|---|---|
| Art field | DNA binding dyes | Hydrolysis probes, oligonucleotides | Hybridization probes, oligonucleotides | The present invention |
| PCR | Good | Good | Good | Good |
| RCA | Moderate | Bad | Good | Good |
| Isothermal polymerization | Moderate | Good | Bad | Good |

In the present invention, the fluorescence material linked-nucleotide serves the dual role of producing fluorescence signal as well as being used as a substrate. Therefore, the present invention is very economically beneficial because it is unnecessary to prepare probes, but can be applied to analyze various real-time polymerase reactions such as PCR, RCA and isothermal polymerization reaction, and shows much improved qualities of performance than the past methods.

### BRIEF DESCRIPTION OF THE DRAWINGS

Fig. 1 is a schematic showing a principle of producing fluorescence signal in case of performing real-time polymerase reaction using the fluorescence material linked-nucleotide.
Fig. 2 is a reaction formula showing a method for synthesizing the fluorescence material linked-nucleotide.
Fig. 3 is a result of PAGE (polyacrylamide gel electrophoresis) for searching a polymerase capable of leading polymerization by using the fluorescence material linked-nucleotide as a substrate.
Fig. 4a and 4b are graphs showing an analysis result of quantitative real-time PCR using the fluorescence material linked-nucleotide and Therminator γ DNA polymerase.
   (DNA concentration in a standard sample is from 10 nM to 10 fM)
Fig. 5a and 5b are graphs showing an analysis result of quantitative real-time PCR using unmodified nucleotide and Taq DNA polymerase.
Fig. 6 is a graph showing an analysis result of real-time RCA using the fluorescence material linked-nucleotide and phi29 DNA polymerase.

### DETAILED DESCRIPTION OF THE EMBODIMENTS

The present invention is further illustrated by the following examples, but the following examples relate to preferred embodiments and are not to be construed to be limiting on the scope of the invention.

### <Example 1>

### Synthesis of NH₂-dGTP

50ul of dGTP (100mM) and 30mg of EDC (N-Ethyl-N'-(3-dimethylaminopropyl) carbodiimide hydrochloride, Sigma-Aldrich) were added to 500ul of MES buffer solution (100mM, Sigma-Aldrich). The solution was stirred for 5 min at room temperature. Then, 10 mg of ethylenediamine·HCl (Sigma-Aldrich) was added in the solution and the solution was stirred for 16 hours at 4 °C. Purified NH₂-dGTP was obtained by using reverse-phase HPLC.

Specifically, in order to perform reverse-phase HPLC, 20 mM TEAB (tetraethylammonium bicarbonate) diluted with water was used as Buffer A and 20 mM TEAB (tetraethylammonium bicarbonate) in 90 % acetonitrile was used as Buffer B. Buffer B was set up to be 0% for 10 min and HPLC was performed while Buffer B was adjusted to be from 0% (10 min) to 100% (40 min).

### <Example 2>

### Synthesis of γF-dGTP

The purified NH₂-dGTP in <example 1> was dissolved in 50 ul of PBS (phosphate buffered saline) to be 5.4 mM and subsequently Bodipy-FL (10 mM, 400 ul) dissolved in DMSO (dimethylsulfoxide) was added thereto. The solution was stirred for 3 hours at room temperature. Purified γF-dGTP was obtained by using reverse-phase HPLC as in <example 1>.

### <Example 3>

### Search of DNA polymerase capable of using γF-dGTP as a substrate

In order to search DNA polymerase, primer extension reaction was performed. Specifically, the primer extension reaction was performed by using 20ul of mixed reaction solution comprising the fluorescence-labeled primer having SED ID: 1 (5'-fluorescein-CTGACTGCATCT**AGACGTGACTGA,** 1 uM, Intergrated DNA Technologies), the template chain having SEQ ID: 2 (5'-AGATGCAGTCAGTCAGTCAGTCAGTCAGTCAGTCAGTCAGTCAGTCAGTCAGTC AGTCAG**TCAGTCACGTCT**, 1 uM, Intergrated DNA Technologies), dATP (25 uM, Promega), dCTP (25 uM, Promega), dTTP (25 uM, Promega), γF-dGTP (25 uM), polymerase reaction buffer solution (New England Biolabs), and 4 units of DNA polymerase (Taq, Vent(exo-), DeepVent (exo-), Bst, or Therminator γ)(purchased from New England Biolabs)

More specifically, the mixed reaction solution was heated by repeating five times a cycle consisting of 20 sec at 94°C, 40 sec at 46°C, and 90 sec at 60°C.

For a control group experiment, Taq DNA polymerase reaction was performed by using dGTP in a natural state instead of γF-dGTP under the same conditions mentioned above.

In addition, primer extension reaction was performed at 37°C for 7.5 min by using Klenow Fragment (exo-)(purchased from New England Biolabs)

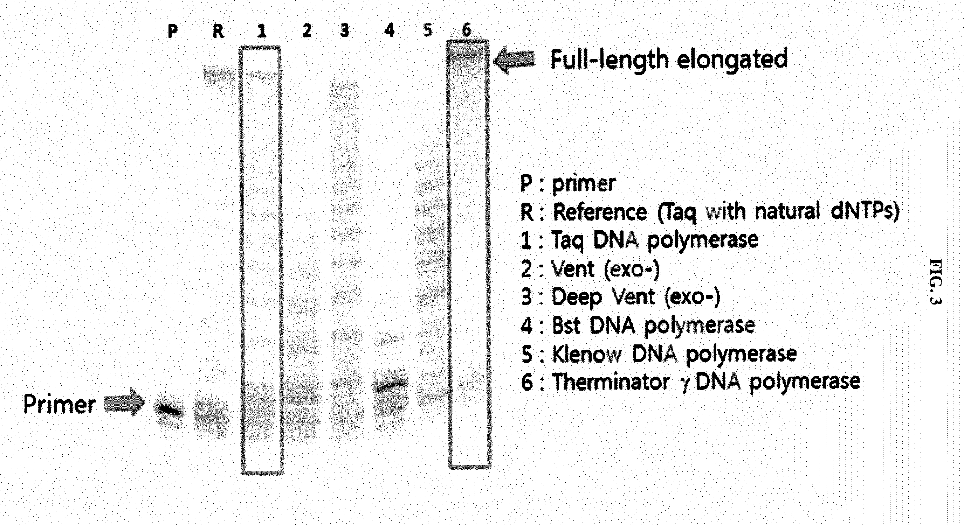

After each polymerase reaction, the mixed reaction solution was developed (Vertical Gel Electrophoresis Unit, Sigma-Aldrich) by using 20% denaturing polyacrylamide gel electrophoresis (PAGE). As a result, an image of DNA fluorescence band was obtained by using scanner (Typhoon9400, GE healthcare). Fig. 3 shows the image of DNA fluorescence band.

As shown in Fig. 3, taq DNA polymerase and therminator γ DNA polymerase can use the modified nucleotide as a substrate, extend DNA template chain, and form a fully extended polymerization product.

### <Example 4>

### Analysis of real-time PCR using γF-dGTP

Real-time PCR analysis was performed by using 20ul of mixed reaction solution comprising the forward primer having SEQ ID: 3 (5'-CCACTCCTCCACCTTTGAC, 100 nM), the reverse primer having SEQ ID: 4 (5'-**ACCCTGTTGCTGTAGCCA**, 100 nM), the template chain having SEQ ID: 5 (5'-CCACTCCTCCACCTTTGCCGCTGGGGCTGGCATTGCCCTCAACGACCACTTTGTC AAGCTCATTTCCTGGTATGCCAACGAATT**TGGCTACAGCAACAGGGT**, from 10 nM to 10 fM), dATP (25 uM, Promega), dCTP (25 uM, Promega), dTTP (25 uM, Promega), γF-dGTP (25 uM), polymerase reaction buffer (New England Biolabs), and Therminator γ DNA polymerase (2 units).

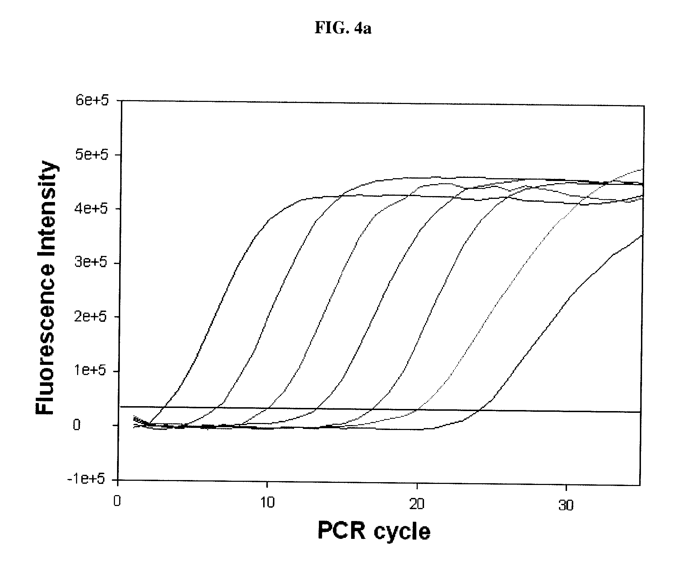

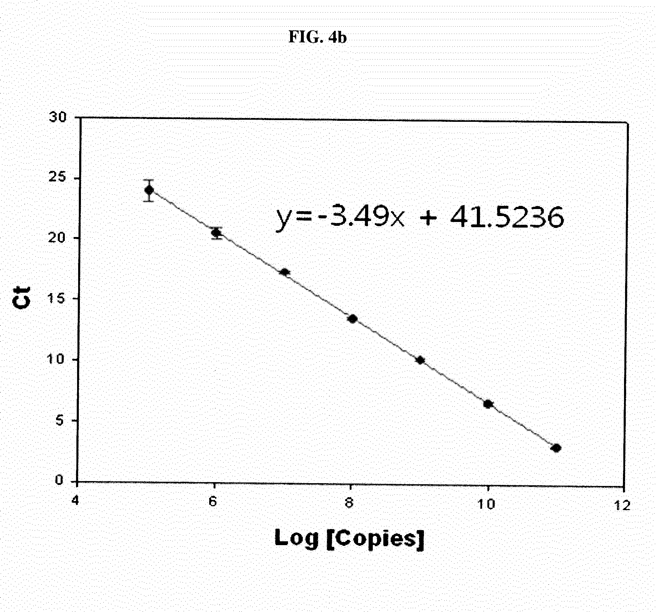

Total of 40 cycles of PCR was performed, wherein each cycle consists of 94°C for 10 sec, 54°C for 15 sec, and 72°C for 10 sec. After each cycle was completed, fluorescence intensity was measured in real time by using StepOne real-time PCR system (ABI). The obtained results were shown Fig. 4a and 4b (Fluorescence filter: ex/em = 490/520 nm).

As shown in Fig. 4a and 4b, total of seven polymerization curves were obtained from the reaction solution in which the template chain was diluted from 10 nm to 10 fm. Threshold was set up by the particular value shown in Fig. 4a and 4b. Ct (threshold cycle) value was set up by determining the point at which the threshold encounters the polymerization curve. Fig. 4a and 4b show functional relation of Ct value and the content of template chain.

### <Comparative example 1>

### Analysis of real-time PCR using SYBR green I

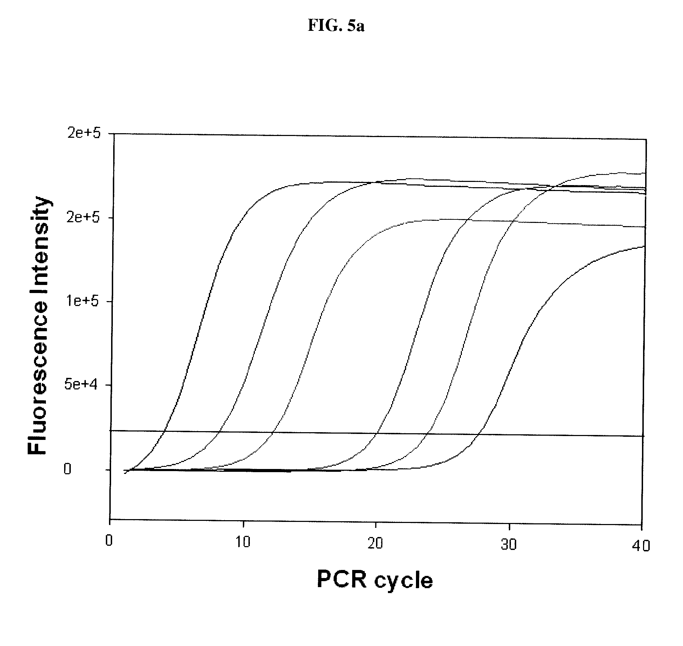

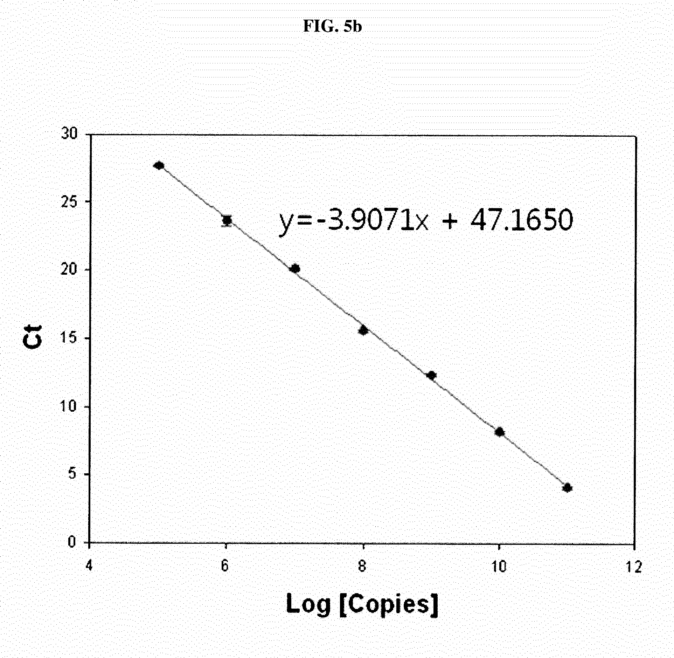

Real-time PCR analysis was performed by using 20ul of mixed reaction solution comprising the forward primer (100 nM) used in <example 4>, the reverse primer (100 nM) used in <example 4>, the template chain (from 10 nM to 10 fM) used in <example 4>, dATP (25 uM, Promega), dCTP (25 uM, Promega), dTTP (25 uM, Promega), dGTP (25 uM, Promega), polymerase reaction buffer (New England Biolabs), SYBR green I (diluted to 1/20,000 , Invitrogen), and Therminator γ DNA polymerase (2 units). Analysis was performed under the PCR conditions, the equipment and the method identical to that of <example 4>. Fig. 5a and 5b show the result.

As shown in Fig. 5a and 5b, real-time PCR results obtained by using γF-dGTP in <example 4> is similar to real-time PCR results obtained by the conventional method. Therefore, it is possible to analyze real-time PCR by using γF-dGTP.

As shown in Table 2, the comparison was made between the results in <example 4> against <comparative example 1>

**[Table 2]**

| Technical item | SYBR green I (comparative example 1) | The present invention (example 4) | Standard |
|---|---|---|---|
| (Efficiency, %) [100×( 10(^{-1/slope)} -1)] | 80.5 | 93.4 | Max = 100% |
| Data Fitting (R² value) | 0.999 | 0.999 | Max = 1.00 |
| Sensitivity (Required PCR cycles for 1 copy number: Y-intercept) | 47.2 | 41.5 | A lower value means more sensitivity. |

(In the above table, any details related to each value may be viewed in http://www3.appliedbiosystems.com/cms/groups/mcb_marketing/documents/generaldocuments/ cms_053906.pdf)

As shown in Table 2, the present invention shows the improved results with regard to a comparative item (efficiency or sensitivity) than the past method using SYBR green I.

### <Example 5>

### Analysis of real-time RCA using γF-dGTP

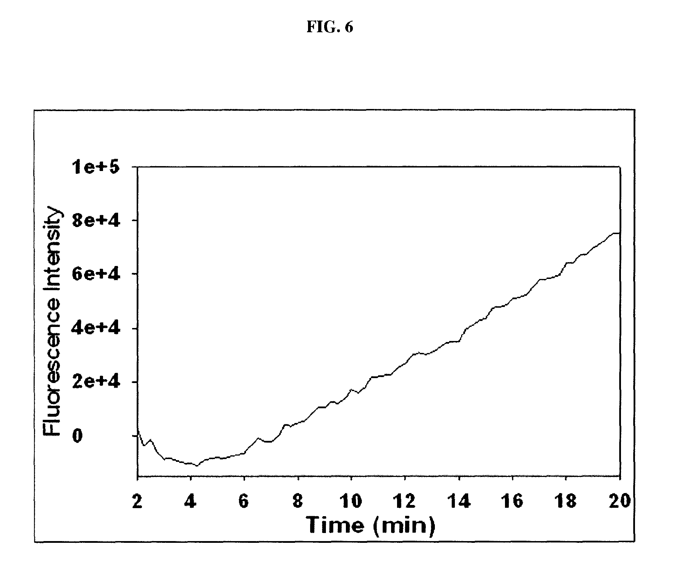

Real-time RCA (rolling circle amplification) analysis was performed by using 20ul of mixed reaction solution comprising the primer having SEQ ID: 6 (5'-CTGCTGCATCTAGACGTGACTGA, 100 nM), the template chain having SEQ ID: 7 (5'-GATGCAGTCAGTCGTCATCGAGTCGTCAGTCAGTCAGTCAGTCAGTCAGTCAGTC AGTCACGTCT, 100 nM), dATP (25 uM, Promega), dCTP (25 uM, Promega), dTTP (25 uM, Promega), γF-dGTP (25 uM), BSA (100 µg/mL), Phi29 DNA polymerase reaction buffer (New England Biolabs), and Phi29 DNA polymerase. (6 units) The reaction was performed at 30°C for 20min and fluorescence intensity at every 20 sec was measured by the equipment used in <example 4>. Fig. 6 shows the result.

As shown in Fig. 6, the fluorescence intensity increases in real-time RCA using the modified nucleotide of the present invention.
## Claims

1. A composition for real-time polymerase reaction comprising a fluorescence material linked-nucleotide represented by Formula (1), and a polymerase which is selected from the group consisting of Taq DNA polymerase and Therminator γ DNA polymerase: 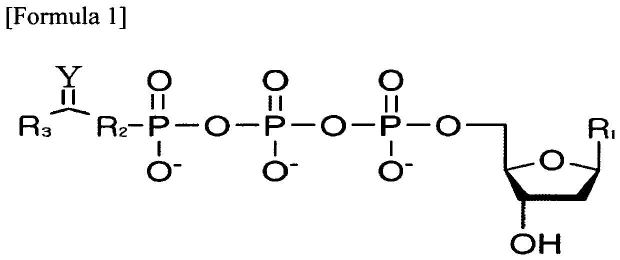
wherein R₁ is an adenine or guanine,
R₂ is -(CH₂OCH₂)m-CH₂NH-, -NHCH₂-(CH₂OCH₂)m-CH₂NH- -(CH₂)n-NH-, or -NH-(CH₂)n-NH-, wherein m is an integer from 1 to 20, and n is an integer from 2 to 60,
R₃ is a fluorescence material, and
Y is O or S.

2. The composition according to claim 1, wherein the composition further comprises dCTP (deoxycytidine triphosphate) and dTTP (deoxythymidine triphosphate).

3. The composition according to claim 1, wherein the fluorescence material is selected from the group consisting of fluorescein, Bodipy-FL, Bodipy-R6G, Pacific Blue, Marina Blue, coumarin, tetramethylrhodamine, Cy5, Cy3, and Texas Red.

4. The composition according to claim 1, wherein the real-time polymerase reaction is selected from the group consisting of real-time PCR (polymerase chain reaction), and isothermal polymerization.

5. An analysis kit for real-time polymerase reaction, comprising the composition for real-time polymerase reaction of any of claims 1 to 4.

6. A method for analyzing real-time polymerase reaction comprising the steps of:
(a) providing the composition for real-time polymerase reaction of any of claims 1 to 4 and primers capable of amplifying a region of target nucleic acid;
(b) extracting nucleic acids from a sample, and performing real-time polymerase reaction by using the composition and the primers provided in step (a); and
(c) analyzing target nucleic acid content in the sample by measuring fluorescence signal in step (b).

7. The method according to claim 6, wherein the real-time polymerase reaction is selected from the group consisting of real-time PCR (polymerase chain reaction), and isothermal polymerization.

## Patentansprüche

1. Zusammensetzung für eine Echtzeit-Polymerasereaktion, die ein mit einem Fluoreszenzmaterial verbundenes Nukleotid, das durch Formel (1) dargestellt wird, und eine aus der aus Taq-DNA-Polymerase und Therminator-γ-DNA-Polymerase bestehenden Gruppe ausgewählte Polymerase umfasst: 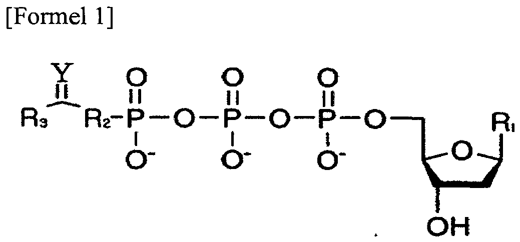
wobei R₁ Adenin oder Guanin ist,
R₂-(CH₂OCH₂)m-CH₂NH-, -NHCH₂-(CH₂OCH₂)m-CH₂NH-, -(CH₂)n-NH- oder -NH-(CH₂)n-NH- ist, wobei m eine ganze Zahl von 1 bis 20 und n eine ganze Zahl von 2 bis 60 ist,
R₃ ein Fluoreszenzmaterial ist und
Y O oder S ist.

2. Zusammensetzung nach Anspruch 1, wobei die Zusammensetzung weiterhin dCTP (Desoxycytidintriphosphat) und dTTP (Desoxythymidintriphosphat) umfasst.

3. Zusammensetzung nach Anspruch 1, wobei das Fluoreszenzmaterial aus der aus Fluorescein, Bodipy-FL, Bodipy-R6G, Pacific Blue, Marina Blue, Cumarin, Tetramethylrhodamin, Cy5, Cy3 und Texas Red bestehenden Gruppe ausgewählt ist.

4. Zusammensetzung nach Anspruch 1, wobei die Echtzeit-Polymerasereaktion aus der aus Echtzeit-PCR (Polymerase-Kettenreaktion) und isothermer Polymerisation bestehenden Gruppe ausgewählt ist.

5. Analyse-Kit für eine Echtzeit-Polymerasereaktion, das die Zusammensetzung für eine Echtzeit-Polymerasereaktion nach einem der Ansprüche 1 bis 4 umfasst.

6. Verfahren zur Analyse einer Echtzeit-Polymerasereaktion, das folgende Schritte umfasst:
(a) Bereitstellen der Zusammensetzung für eine Echtzeit-Polymerasereaktion nach einem der Ansprüche 1 bis 4 und von Primem, die eine Region einer Zielnukleinsäure amplifizieren können;
(b) Extrahieren von Nukleinsäuren aus einer Probe und Durchführen einer Echtzeit-Polymerasereaktion unter Verwendung der in Schritt (a) bereitgestellten Zusammensetzung und Primer; und
(c) Analysieren des Gehalts an Zielnukleinsäure in der Probe durch Messen eines Fluoreszenzsignals in Schritt (b).

7. Verfahren nach Anspruch 6, wobei die Echtzeit-Polymerasereaktion aus der aus Echtzeit-PCR (Polymerase-Kettenreaktion) und isothermer Polymerisation bestehenden Gruppe ausgewählt ist.

## Revendications

1. Composition pour une réaction par polymérase en temps réel comprenant un nucléotide lié à une substance fluorescente représenté par la Formule (1) et une polymérase qui est sélectionnée dans le groupe constitué par l'ADN polymérase Taq et l'ADN polymérase γ Therminator : 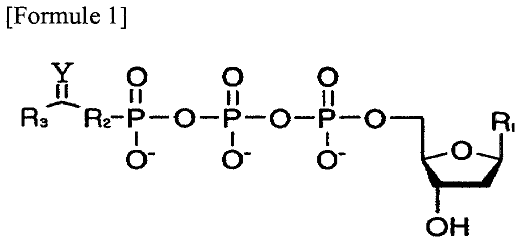
dans laquelle R₁ est l'adénine ou la guanine,
R₂ est -(CH₂OCH₂)m-CH₂NH-, -NHCH₂-(CH₂OCH₂)m-CH₂NH-, -(CH₂)n-NH- ou -NH-(CH₂)n-NH-, dans lesquels m est un nombre entier de 1 à 20 et n est un nombre entier de 2 à 60,
R₃ est une substance fluorescente et
Y est O ou S.

2. Composition selon la revendication 1, dans laquelle la composition comprend en outre de la dCTP (désoxycytidine triphosphate) et de la dTTP (désoxythymidine triphosphate).

3. Composition selon la revendication 1, dans laquelle la substance fluorescente est sélectionnée dans le groupe constitué par la fluorescéine, le Bodipy-FL, le Bodipy-R6G, le Pacific Blue, le Marina Blue, la coumarine, la tétraméthylrhodamine, la Cy5, la Cy3 et le Texas Red.

4. Composition selon la revendication 1, dans laquelle la réaction par polymérase en temps réel est sélectionnée dans le groupe constitué par la PCR en temps réel (réaction en chaîne par polymérase) et la polymérisation isotherme.

5. Trousse d'analyse pour une réaction par polymérase en temps réel comprenant la composition pour une réaction par polymérase en temps réel selon l'une quelconque des revendications 1 à 4.

6. Procédé pour analyser une réaction par polymérase en temps réel comprenant les étapes de :
(a) fournir la composition pour une réaction par polymérase en temps réel selon l'une quelconque des revendications 1 à 4 et des amorces capables d'amplifier une région d'un acide nucléique cible ;
(b) extraire des acides nucléiques d'un échantillon et effectuer une réaction par polymérase en temps réel en utilisant la composition et les amorces fournies dans l'étape (a) ; et
(c) analyser la teneur en acides nucléiques cibles dans l'échantillon en mesurant un signal de fluorescence dans l'étape (b).

7. Procédé selon la revendication 6, dans lequel la réaction par polymérase en temps réel est sélectionnée dans le groupe constitué par la PCR en temps réel (réaction en chaîne par polymérase) et la polymérisation isotherme.


<110> Korea Institute of science and Technology
<120> Modified nucleotides and real-time polymerase reaction using the same
<130> OPP20110132EP
<160> 7
<170> KopatentIn 1.71
<210> 1
   <211> 24
   <212> DNA
   <213> Artificial sequence
<220>
   <223> Primer for searching DNA polymerase
<400> 1
   ctgactgcat ctagacgtga ctga 24
<210> 2
   <211> 72
   <212> DNA
   <213> Artificial Sequence
<220>
   <223> DNA Template for searching DNA polymerase
<400> 2
   agatgcagtc agtcagtcag tcagtcagtc agtcagtcag tcagtcagtc agtcagtcag 60
   tcagtcacgt ct 72
<210> 3
   <211> 19
   <212> DNA
   <213> Artificial sequence
<220>
   <223> Forward primer for real-time PCR
<400> 3
   ccactcctcc acctttgac 19
<210> 4
   <211> 18
   <212> DNA
   <213> Artificial Sequence
<220>
   <223> Reverse primer for real-time PCR
<400> 4
   accctgttgc tgtagcca 18
<210> 5
   <211> 102
   <212> DNA
   <213> Artificial sequence
<220>
   <223> DNA template for real-time PCR
<400> 5
   ccactcctcc acctttgccg ctggggctgg cattgccctc aacgaccact ttgtcaagct 60
   catttcctgg tatgccaacg aatttggcta cagcaacagg gt 102
<210> 6
   <211> 23
   <212> DNA
   <213> Artificial sequence
<220>
   <223> Primer for real-time RCA
<400> 6
   ctgctgcatc tagacgtgac tga 23
<210> 7
   <211> 65
   <212> DNA
   <213> Artificial sequence
<220>
   <223> DNA template for real-time RCA
<400> 7
   gatgcagtca gtcgtcatcg agtcgtcagt cagtcagtca gtcagtcagt cagtcagtca 60
   cgtct 65